Europäisches Patentamt

European Patent Office

Office européen des brevets

(11) Publication number: **0 255 291 B1**

# EUROPEAN PATENT SPECIFICATION

(45) Date of publication of patent specification: **24.06.92**  (51) Int. Cl.⁵: **G01N  27/42**, C12M 1/40

(21) Application number: **87306513.0**

(22) Date of filing: **23.07.87**

Divisional application 91117022.3 filed on 23/07/87.

(54) Method and apparatus for electrochemical measurements.

(30) Priority: **23.07.86 GB 8618022**

(43) Date of publication of application:
**03.02.88 Bulletin  88/05**

(45) Publication of the grant of the patent:
**24.06.92 Bulletin  92/26**

(84) Designated Contracting States:
**AT BE CH DE ES FR GB GR IT LI NL SE**

(56) References cited:
**EP-A- 0 078 636
EP-A- 0 125 137
EP-A- 0 177 743
DE-B- 1 932 581**

**AGRICULTURAL AND BIOLOGICAL CHEMIS-
TRY, vol. 48, no. 8, August 1984, pages
1969-1976, Tokyo, JP; T. IKEDA et al.:
"Electrocatalysis with a gluose-
oxidase-immobilized graphite electrode"**

(73) Proprietor: **UNILEVER PLC
Unilever House Blackfriars P.O. Box 68
London EC4P 4BO(GB)**

(84) Designated Contracting States:
**GB**

(73) Proprietor: **UNILEVER NV
Burgemeester s'Jacobplein 1 P.O. Box 760
NL-3000 DK Rotterdam(NL)**

(84) Designated Contracting States:
**BE CH DE ES FR GR IT LI NL SE AT**

(72) Inventor: **Birch, Brian Jeffrey
14 Duchy Close
Chelveston Northamptonshire, NN9
6AW(GB)**
Inventor: **Burns, Ian William
The Little House Shelton
Huntingdon Cambridgeshire PE18 0NP(GB)**

(74) Representative: **Butler, David John et al
Unilever PLC Patents Division P.O. Box 68
Unilever House
London EC4P 4BO(GB)**

ANALYTICAL CHEMISTRY, vol. 56, no. 2, February 1984, pages 148-152, Easton, Pennsylvania, US; H. DURLIAT et al.: "Amperometric enzyme electrode for determination of glucose based on thin-layer spectroelectrochemistry of glucose oxidase"

ANALYTICAL CHEMISTRY, vol. 50, no. 7, June 1978, pages 944-950, Easton, Pennsylvania, US; R.E. ADAMS et al.: "Coulometric flow analyzer for use with immobilized enzyme reactors"

INDUSTRIAL LABORATORY, vol. 46, no. 2, February 1980, pages 116-118, New York, US; T.K. KHAMRAKULOV et al.: "Coulometric determination of chlorine"

JOURNAL OF PHYSICS E. SCIENTIFIC INSTRUMENTS, vol. 18, no. 9, September 1985, pages 736-749, Bristol, GB; C. NYLANDER "Chemical and biological sensors"

## Description

### Field of invention

This invention relates to methods and apparatus for making electrochemical measurements, in particular but not exclusively for the purpose of carrying out microchemical testing on small liquid samples of biological, e.g. clinical origin.

### Background to the Invention

F. Schlapfer et al (Clin.Chim.Acta, 1974, pp 283-289) described electrochemical measurement of glucose concentration using glucose oxidase and soluble electron transfer substances such as ferricyanide, p-benzoquinone, 2,6-dichlorophenolindophenol, pyocyanine, thionine or methylene blue, interacting with amperometric noble-metal electrodes.

These arrangements have not given rise to glucose-measurement products which are simple and convenient to use in environments far removed from the skilled inhabitants of analytical laboratories.

Since 1974 a variety of further electrode arrangements have been proposed for chemical/immunochemical analysis, among them electrodes carrying immobilised redox mediators as well as enzymes, for example EP 0 078 636, 0 125 136 and 0 125 139 (Genetics International), and 0 142 301 (Serono). EP 0 177 743 (Shimadzu) describes enzyme electrodes of somewhat complex construction, which are capable of use to measure a number of enzyme substrates by amperometry, using electron transfer mediators.

EP 0 125 137 discloses an electrode sensing system, for monitoring components in a liquid mixture, using a probe-type sensor which typically comprises an electrode surface carrying a generally insoluble electron transfer mediator, in turn coated with an enzyme, the electron transfer mediator acting to transfer charge between the enzyme and the electrode. The system is typically used to determine glucose concentrations, using amperometric techniques.

The paper in "Analytical Chemistry", Volume 56, No 2, February 1984, pages 148 to 152 discloses an enzyme electrode using a thin layer platinum electrode in conjunction with the enzyme glucose oxidase or flavine adenine dinucleotide, and refers to the possibility of reducing the depth of the reaction chamber to optimize performance.

This invention aims to provide measurement apparatus and methods to enable quick, convenient and accurate measurement of various constituents of liquid samples, especially of biological origin, e.g. clinical samples of blood, serum, or urine.

The invention also aims to provide measurement apparatus of simple construction which can be treated as disposables.

It is also an aim of the invention to enable convenient and in many cases quantitative electrochemical methods to be applied to analytes which do not themselves react with electrodes.

It is a further aim of the present invention to provide electrochemical apparatus for convenient liquid sample analysis but without requiring complex electrode structures involving immobilised components.

### Summary of the invention

According to one aspect of the invention there is provided a method for measurement or detection of a component of an aqueous liquid sample, said method comprising:

placing a sample, possibly containing the component of interest, into a capillary fill reaction cell, said reaction cell comprising at least two electrodes adapted to coulometric measurements, so that the sample forms a layer of liquid having a thickness less than about 0.2 millimeter in a reaction zone overlying one of said electrodes,

reacting said component, if present, directly or indirectly with a redox reagent initially present in the form of a solid releasable layer deposited on an internal surface of the reaction cell and which is released into solution in said sample in said zone, thereby to form in said zone in a quantity proportional to the amount of said component present in said zone of an electrochemically oxidizable or reducible substance capable of reacting electrochemically with said electrode, and being different from said redox reagent, and

coulometrically detecting or estimating a quantity of electrical charge which relates to said oxidizable or reducible substance in said zone to provide an indication of the amount of said component of interest.

The component to be analysed may oxidise or reduce directly or indirectly an electron transfer substance which is then estimated electrochemically. The production of the electrochemically oxidisable or reducible substance may be made to occur enzymatically.

The result relating to the quantity of the substance to be measured can be of use, among other things, as an index of the concentration of the substance in a liquid sample.

In this specification and claims 'redox reagent' and similar terms and 'electron transfer reagent' and corresponding terms are mutually inclusive.

Also provided by the invention is apparatus for carrying out electrochemical detection or measurement of a component of an aqueous liquid sample, said apparatus comprising:

a capillary fill reaction cell comprising at least two electrodes, the electrodes being adapted to coulometric measurements and being located in or adjacent to a reaction zone, said reaction zone being capable of receiving an aqueous liquid sample possibly containing said component of interest, and said electrodes and said reaction zone being arranged so that liquid in said zone contacts said electrodes and forms a layer having a thickness less than about 0.2 millimeter overlying one of said electrodes,

and said cell also comprising a redox reagent in the form of a solid releasable layer deposited on an internal surface of the cell and located to contact said sample when said sample is introduced into said cell and into said reaction zone, said redox agent being released into solution in said aqueous sample in said zone and reacting directly or indirectly with said component to form an electrochemically oxidizable or reducible substance in said zone, said oxidizable or reducible substance being capable of reacting electrochemically with said electrode and being different from said redox reagent.

It is especially preferred to provide in the use of this method a cell which confines the liquid reagents to react with the electrode to a sufficiently thin layer overlying the electrode to permit coulometric measurement of the electro-active material to take place in a short time. A suitable thickness for the liquid layer is for example of the order of about 0.02 to 0.2 mm, for example about 0.1 mm. Capillary-fill cells with a configuration as described in EP 0 170 375 (Unilever) are among the cells suitable in this respect. In certain useful arrangements within the scope of the invention, the cell may confine a defined reactive volume of sample or reaction liquid in a space of defined width between a cell wall and an electrode of defined area. Liquid outside the volume may be able to diffuse inwards but for example only at an inappreciable rate compared to the time required for reaction of the liquid in the defined volume. In other useful embodiments, the cell may define a volume of liquid to provide material to react at the electrode.

In one preferred kind of test arrangement, the component of the aqueous liquid sample to be measured is a reducible sugar such as glucose, and a reagent with which said sugar is allowed to react is an oxidase enzyme specific for said sugar desired to be measured, such as glucose oxidase, together with any further substrate. Glucose and glucose oxidase together react with an electron transfer mediator such as ferricyanide ion in place of their normal further substrate oxygen to produce a corresponding quantity of ferrocyanide ion. Finally the ferrocyanide ion so produced is estimated coulometrically by anodic oxidation.

Among further suitable examples of electron transfer substances are methylene blue, p-benzoquinone, 2,6-dichlorophenolinophenol, pyocyanine and thionine.

The component to be measured can in general be formed by initial enzymic or chemical conversion of an analyte: e.g. an analyte can be sucrose, and invertase can be contained in said cell to form from said sucrose glucose by hydrolysis: the glucose so formed can then be measured by the methods described herein.

An oxidoreductase enzyme can be present in said cell to mediate any desired reaction between the component to be measured and any additional electron transfer reagent.

In one class of tests which can be carried out using the devices and methods described in more detail herein, the component to be measured comprises an electrochemically reducible metal ion or an electrochemically oxidisable inorganic ion or an electrochemically oxidisable or reducible organic compound and is measured either by direct coulometry or by coulometry after initial electrochemical conversion to an electrochemically oxidisable or reducible intermediate. It may not in all cases be necessary to deplete completely the electroactive species to be measured.

The form of the reaction cell in which these reactions are allowed to take place can contribute significantly to the convenience of the test procedure. It is preferred to use an adapted form of the capillary fill cells provided with electrodes as described in European Specification No 0 170 375 (Unilever), containing electrodes of suitable impedance carried as thin films on one or more walls thereof. The drawings and description of said specification are incorporated herein by reference, to be modified by the indications given herein for making and using the measurement devices and methods of the present invention.

Such a cell as adapted for the purposes of the present invention can suitably for example comprise three electrodes, viz (a) a working electrode, for example of gold or other noble metal, carbon or graphite in any convenient form, e.g. wax-impregnated graphite; (b) a counterelectrode, chosen from a similar range of materials as given for electrode (a), and possibly of the same material as electrode (a) itself; and (c) a reference electrode, for example a silver/chloride electrode, or pH electrode.

Choice of electrode materials can for many purposes preferably be made among gold, silver and carbon film electrodes.

In one convenient form, both or all electrodes are contained as films in a capillary fill cell formed between two parallel flat plates spaced apart by about 0.1 mm cell thickness, with about 0.1 mm thick tracks of sealing material forming the remaining sides of the cell apart from an aperture for entry of liquids.

It can be convenient to form such a cell using opposed plates of for example ceramic, plastics or glass. When such a cell is fabricated, as is preferred, by the use of ceramics, a suitable substrate can be for example a 96% alumina substrate (Kyocera A4476 - Trade Mark), and a preferred material for the electrodes to be formed thereon is gold, applied as gold printing paste (Engelhard T4474 - Trade Mark), to be applied in a high temperature oxidative furnace in accordance with the ordinary methods of use of that material. This results in the context of this invention in a gold layer with overlying thin oxide layer capable of constituting a highly reproducible electrode. Whenever desired, part of the metal layer can be blanked off by overlying dielectric layers e.g. formed of dielectric printing ink (DuPont 5704 - Trade Mark) applied to the substrate according to the ordinary manner of use of that material.

When reactions of the kinds described above are allowed to occur in a cell as described above, it is found that a working electrode can easily deplete substantially all of the electroactive material in that part of the liquid that overlies the working electrode, before any substantial lateral diffusion has taken place.

Accordingly, it is preferred to use cells of such dimensions that this situation prevails: i.e. that the time required for lateral diffusion of an appreciable amount of reactive material from the region outside that which overlies the working electrode, to the region overlying the working electrode, is much longer than the time required for diffusion of cell contents across the thickness of the cell and for depletion by an electrode of the material capable of reacting with it from the region of the cell overlying said electrode.

An advantage arising from use of the invention in this manner is that the measurement can be made substantially insensitive to the nature of the electrode material and calibration of the measurements can be particularly simple and uniform as between samples of the devices as described herein.

The arrangements of the invention can for example take the form of coulometric measurements. Such measurement methods are in themselves known and their details do not constitute the present invention.

Further details are given below in connection with the following illustrative example.

Example

Examples of glucose measurement will now be described non-limitatively, first in a coulometric embodiment.

Reagents for the test can conveniently be dried down on to a surface which either forms part, or will form part, of a glass or ceramic inner surface of a capillary fill cell.

The reagents can be dried down either by filling reagent liquid into a pre-formed cell (e.g. 0.1 mm wide) and then drying, or by screen-printing a liquid layer up to 0.1 mm thick to be dried on to said surface which will form part of said cell when said cell is fabricated from a component carrying dried printed reagents.

In the present example the reagents are chosen so that upon rehydration in the sample liquid filling the cell they give:-
buffer (preferably about 0.1M ammonium citrate, otherwise e.g. 0.5M sodium phosphate) adjusted to approximtely neutral pH;
0.5M potassium ferricyanide; and
0.5 mg/ml glucose oxidase (a considerable excess, which may be reduced).

Low molecular weight (about 40,000) polyvinylpyrrolidone can be used as a carrier and/or stabiliser, used in a quantity and concentration dictated largely by the volume of reagent liquid to be applied and dried, and by the method of application, e.g. at 5% w/v in liquid to be filled and dried in a preformed cell, and at higher concentration (optionally lower volume) in liquid to be printed. In other variants, any other reagents can also be present to suit the test to the test sample liquids to be used - e.g. further anticoagulant besides citrate, if necessary, where whole blood is to be tested. This example gives a sensitivity range of about 0 to 20 mmolar glucose concentration. In other variant examples, suitable concentrations for the ferricyanide lie in the order of about 3 times the maximum concentration of glucose to be estimated. Chloride ion should be present where a chloride electrode is used as the reference electrode. Also usefully present in certain variants can be an inhibitor of catalase, e.g. sodium azide, and/or a chemical deoxygenator.

The dried reagents may be carried and/or stabilised on a surface by inclusion of a water-soluble polymer, e.g. polyvinylpyrrolidone, or alternatively a water-insoluble polymer support such as a thin layer of cellulose acetate.

Test liquid is introduced into the cell. The immobilised reagents including the electron transfer substance (ferricyanide) are allowed to dissolve and disperse throughout the volume of the test liquid, and the reaction of the glucose and the glucose oxidase is allowed to take place, reducing the ferricyanide to ferrocyanide.

The electrical arrangements can comprise for example a conventional potentiostatic control arrangement in which, for example, a voltage-follower impedance transformer is connected with its input taken from the working electrode and reference electrode, and its output taken, through circuitry to apply a working low-impedance voltage between the working electrode and the counterelectrode, in a negative feedback arrangement such that the p.d. between the working electrode and reference electrode is kept close to a desired level.

A current integrator is connected with its inputs taken from the working electrode and counterelectrode, and delivers as its output a signal which is to be taken as the coulometric measurement given by the device.

Initially, the potentiostatic control is set so that the p.d. between working and reference electrode is insufficient to allow electrode reaction of the form of electron transfer substance produced indirectly by reaction of the analyte: at a point in time from which the coulometric integration is to be started, the voltage is stepped to a level that does allow such electrode reaction. Typically, a potential at the working electrode is chosen that oversteps the redox potential of the electron transfer material by of the order of about 0.05 - 0.1 volt, to maximise the wanted reaction relative to any side reactions. Then the current is monitored and for example integrated for a wanted appropriate interval of time to provide the desired signal indicative of the wanted measurement. Suitable 'inactive' and 'active' potentials for the ferro/ferricyanide embodiments can be for example in the range up to about + 0.25 volt and + 0.5 volt respectively.

A preferred configuration for this and other examples involves the use of a cell comprising a pair of gold electrodes. In this case one gold electrode can serve as a counter-electrode as well as a reference electrode, and a substantially invariant potential can be obtained via the ferricyamide present in the reaction mixture, of which the quantity can most suitably be large, (e.g. much larger than the quantity of analyte and ferrocyamide formed by reduction,) hence substantially constant. A preferred operating potential can be at about + 0.15 volt.

It can be especially convenient to provide a simple combination of potentiostat and digital meter readout for the integrator. Then the user can watch until the digital reading comes substantially to a standstill (i.e. upon completion of the electrode reaction) and takes the reading at that point as the wanted measurement result, or in another arrangement, involving automatic data processing, the digital signal can be stored when its rate of change has subsided below a preset threshold rate.

It is found that typical electrode currents in this coulometry are of the order of fractions of a milliamp for a few minutes where typical blood glucose concentrations are measured, e.g. in whole blood, plasma or serum.

In one alternative variant of this embodiment, there may be no 'inactive' potential applied to the cell formed by the working and counter electrodes, but rather this cell may be left open-circuit until the current integration is to take place.

In a variant of the process mercuric ions can be measured using a gold electrode by introducing a sample into a capillary fill cell, and estimating a trace quantity of mercury by step coulometry. This comprises holding the electrode potential more positive than the mercuric ion reduction potential, e.g. + 0.5 volt, until other current-generating processess have decayed. Then the voltage is stepped to a value more negative than the mercuric ion reduction potential, e.g. to -0.1 volt. Then the charge passed until the current decays can be taken as an index of the mount of mercury present.

In further embodiments, it can be sufficient to use a working electrode and a further electrode combining the functions of reference and counter electrode, provided that the further electrode, is a metal/metal-halide reference electrode and the corresponding halide is present in the sample, preferably at standardised concentration. Alternatively, any other further electrode of low electrochemical impedance and adequately-defined potential may be used. Suitable electrodes for use are for example as described in EP 0 186 286 (Unilever).

The invention described herein is susceptible of many modifications and variations as will be apparent to the skilled reader, and the disclosure herein extends to the use of all combinations and subcombinations of the features as claimed.

**Claims**

1.  A method for measurement or detection of a component of an aqueous liquid sample, said method comprising:

    placing a sample, possibly containing the component of interest, into a capillary fill reaction cell, said reaction cell comprising at least two electrodes adapted to coulometric measurements, so that the sample forms a layer of liquid having a thickness less than about 0.2 millimeter in a reaction zone overlying one of said electrodes,

    reacting said component, if present, directly or indirectly with a redox reagent initially present in the form of a solid releasable layer deposited on an internal surface of the reaction cell and which is released into solution in said sample in said zone, thereby to form in said zone a quantity proportional to the amount of said component present in said zone of an electrochemically oxidizable or reducible substance capable of reacting electrochemically with said electrode, and being different from said redox reagent, and

coulometrically detecting or estimating a quantity of electrical charge which relates to said oxidizable or reducible substance in said zone to provide an indication of the amount of said component of interest.

2. A method according to claim 1, wherein said component to be measured is formed by initial enzymatic or chemical conversion of an analyte.

3. A method according to claim 1 or 2, wherein said redox reagent is selected from the group comprising ferricyanide, methylene blue, p-benzoquinone, 2,6-dichlorophenolindophenol, pyocyanine and thionine, and said electrochemically oxidizable or reducible substance is oxidizable and comprises a reduced form of said redox reagent.

4. A method according to any one of claims 1 to 3, wherein said component is reacted with an enzyme and said redox reagent thereby to form said electrochemically oxidizable or reducible substance.

5. A method according to claim 4, wherein said enzyme comprises an oxidoreductase enzyme.

6. A method according to claim 4 or 5, wherein said component comprises a reducing sugar and said enzyme comprises a corresponding sugar oxidase.

7. A method according to claim 6, wherein said component comprises glucose and said enzyme comprises glucose oxidase.

8. Apparatus for carrying out electrochemical detection or measurement of a component of an aqueous liquid sample, said apparatus comprising:

a capillary fill reaction cell comprising at least two electrodes, the electrodes being adapted to coulometric measurements and being located in or adjacent to a reaction zone, said reaction zone being capable of receiving an aqueous liquid sample possibly containing said component of interest, and said electrodes and said reaction zone being arranged so that liquid in said zone contacts said electrodes and forms a layer having a thickness less than about 0.2 millimeter overlying one of said electrodes,

and said cell also comprising a redox reagent in the form of a solid releasable layer deposited on an internal surface of the cell and located to contact said sample when said sample is introduced into said cell and into said reaction zone, said redox reagent being released into solution in said aqueous sample in said zone and reacting directly or indirectly with said component to form an electrochemically oxidizable or reducible substance in said zone, said oxidizable or reducible substance being capable of reacting electrochemically with said electrode and being different from said redox reagent.

9. Apparatus according to claim 8, further comprising an enzyme located to contact said sample when said sample is introduced into said cell and into said reaction zone, said enzyme being capable of catalysing direct or indirect reaction between said component and said redox reagent.

10. Apparatus according to claim 9, wherein said enzyme is carried as a releasable layer on a surface of said reaction cell or reaction zone which in use is contacted by aqueous sample introduced into said apparatus.

11. Apparatus according to claim 9 or 10, wherein said enzyme comprises an oxidoreductase.

12. Apparatus according to claim 11, wherein said enzyme comprises glucose oxidase.

13. Apparatus according to any one of claims 8 to 12, wherein said redox reagent is selected from the group comprising ferricyanide, methylene blue, p-benzoquinone, 2,6-dichlorophenolindophenol, pyocyanine and thionine, and said electrochemically oxidizable or reducible substance is oxidizable and comprises a reduced form of said redox reagent.

14. Apparatus according to any one of claims 8 to 13, wherein said electrodes are selected from the group comprising gold, carbon and silver electrodes.

**Revendications**

1. Procédé de mesure ou de détection d'un composant d'un échantillon liquide aqueux, ledit procédé consistant à:

placer un échantillon, contenant éventuellement le composant intéressant, dans une cellule à réaction à remplissage capillaire, ladite cellule à réaction comprenant au moins deux électrodes adaptées aux mesures coulométriques, afin que l'échantillon forme une couche de liquide ayant une épaisseur inférieure à environ 0,2 millimètre dans une zone réactionnelle recouvrant une desdites électrodes,

à faire réagir ledit composant, si présent, directement ou indirectement avec un réactif redox initialement présent sous la forme d'une couche solide libérable déposée sur une surface interne de la cellule réactionnelle et qui est libéré dans la solution dans ledit échantillon dans ladite zone, pour former de cette façon dans ladite zone une quantité proportionnelle à la quantité dudit composant présent dans ladite zone d'une substance oxydable ou réductible électrochimiquement capable de réagir électrochimiquement avec ladite électrode, et étant différente dudit réactif redox, et

à détecter ou à estimer coulométriquement une quantité de charge électrique qui est fonction de ladite substance oxydable ou réductible dans ladite zone pour fournir une indication de la quantité dudit composant intéressant.

2. Procédé selon la revendication 1, dans lequel ledit composant à mesurer est formé par la conversion enzymatique ou chimique initiale d'un analyte.

3. Procédé selon la revendication 1 ou 2, dans lequel ledit réactif redox est choisi parmi le groupe constitué de ferricyanure, du bleu de méthylène, du p-benzoquinone, du 2,6-dichlorophénolindophénol, de la pyocyanine et de la thionine, et ladite substance oxydable ou réductible électrochimiquement est oxydable et comprend une forme réduite dudit réactif redox.

4. Procédé selon l'une quelconque des revendications 1 à 3, dans lequel ledit composant est mis à réagir avec une enzyme et ledit réactif redox pour former de cette façon ladite substance oxydable ou réductible électrochimiquement.

5. Procédé selon la revendication 4, dans lequel ladite enzyme comprend une enzyme oxydoréductase.

6. Procédé selon la revendication 4 ou 5, dans lequel ledit composant comprend un sucre réducteur et ladite enzyme comprend un sucre oxydase correspondant.

7. Procédé selon la revendication 6, dans lequel ledit composant comprend le glucose et ladite enzyme comprenant la glucose oxydase.

8. Appareil pour réaliser la détection ou la mesure électrochimique d'un composant d'un échantillon liquide aqueux, ledit appareil étant constitué de:

une cellule à réaction à remplissage capillaire comprenant au moins deux électrodes, les électrodes étant adaptées aux mesures coulométriques et étant situées dans ou, adjacentes à une zone réactionnelle, ladite zone réactionnelle étant capable de recevoir un échantillon liquide aqueux contenant éventuellement ledit composant intéressant, et lesdites électrodes et ladite zone réactionnelle étant disposées de façon que le liquide dans ladite zone soit en contact avec lesdites électrodes et forme une couche ayant une épaisseur inférieure à environ 0,2 millimètre s'étendant sur une desdites électrodes,

et ladite cellule comprenant également un réactif redox sous la forme d'une couche solide libérable déposée sur une surface interne de la cellule et située au contact dudit échantillon quand ledit échantillon est introduit dans ladite cellule et à l'intérieur de ladite zone réactionnelle, ledit réactif redox étant libéré dans la solution dans ledit échantillon aqueux dans ladite zone étant mis à réagir directement ou indirectement avec ledit composant pour former une substance oxydable ou réductible électrochimiquement dans ladite zone, ladite substance oxydable ou réductible étant capable de réagir électrochlmiquement avec ladite électrode et étant différente dudit réactif redox.

9. Appareil selon la revendication 8, comprenant de plus une enzyme située au contact dudit échantillon quand ledit échantillon est introduit dans ladite cellule et à l'intérieur de ladite zone réactionnelle, ladite enzyme étant capable de catalyser directement ou indirectement la réaction entre ledit composant et ledit réactif redox.

10. Appareil selon la revendication 9, dans lequel ladite enzyme est portée comme une couche libérable sur une surface de ladite cellule à réaction ou zone réactionnelle qui en utilisation est en contact avec l'échantillon aqueux introduit dans ledit appareil.

11. Appareil selon la revendication 9 ou 10, dans lequel ladite enzyme comprend une oxydoréductase.

12. Appareil selon la revendication 11, dans lequel ladite enzyme comprend la glucose oxydase.

13. Appareil selon l'une quelconque des revendications 8 à 12, dans lequel ledit réactif redox est choisi parmi le groupe constitué de ferricyanure, du bleu de méthylène, du benzoquinone, du 2,6-dichlorophénolindophénol, de la pyocyanine et de la thionine, et ladite substance oxydable ou réductible électrochimiquement est oxydable et comprend une forme réduite dudit réactif redox.

14. Appareil selon l'une quelconque des revendications 8 à 13, dans lequel lesdites électrodes sont choisies parmi le groupe constitué d'électrodes en or, en carbone et en argent.

## Patentansprüche

1. Verfahren zum Messen oder Feststellen einer Komponente einer wäßrigen Flüssigkeitsprobe, das umfaßt:

das Einführen einer Probe, die die interessierende Komponente möglicherweise enthält, in eine Reaktionszelle zur kapillaren Füllung, wobei die Reaktionszelle mindestens zwei für coulometrische Messungen geeignete Elektroden umfaßt, so daß die Probe eine Füssigkeitsschicht einer Dicke von weniger als etwa 0.2 mm in einer Reaktionzone bildet, die über einer der Elektroden liegt:

das direkte oder indirekte Umsetzen dieser gegebenenfalls vorliegenden Komponente mit einem Redox-Reagenz, das anfänglich in Form einer festen, freisetzbaren Schicht vorliegt, die auf einer inneren Oberfläche der Reaktionszelle abgeschieden ist und in die Lösung in der Probe in der Zone freigesetzt wird, wodurch in dieser Zone in einer Menge, die der Menge dieser in der Zone vorliegenden Komponente Proportional ist, eine elektrochemisch oxidierbare oder reduzierbare Substanz gebildet wird, die elektrochemisch mit der Elektrode reagieren kann und von dem Redox-Reagenz verschieden ist, und

das coulometrische Feststellen oder Bestimmen einer Menge an elektrischer Ladung, die sich auf die oxidierbare oder reduzierbare Substanz in dieser Zone bezieht, um so ein Anzeichen für die Menge der interessierenden Komponente zu ergeben.

2. Verfahren gemäß Anspruch 1, worin die zu messende Komponente durch anfängliche enzymatische oder chemische Umwandlung eines Analyten gebildet wird.

3. Verfahren gemäß Anspruch 1 oder 2, worin das Redox-Reagenz aus der aus Cyanoferrat-(III), Methylenblau, p-Benzochinon, 2,6-Dichlorphenolindophenol, Pyocyanin und Thionin bestehenden Gruppe ausgewählt wird und die elektrochemisch oxidierbare oder reduzierbare Substanz oxidierbar ist und eine reduzierte Form des Redox-Reagenzes umfaßt.

4. Verfahren gemäß irgendeinem der Ansprüche 1 bis 3, worin die Komponente mit einem Enzym und dem Redox-Reagenz umgesetzt wird, um so die elektrochemisch oxidierbare oder reduzierbare Substanz zu bilden.

5. Verfahren gemäß Anspruch 4, worin das Enzym ein Oxidoreduktaseenzym umfaßt.

6. Verfahren gemäß Anspruch 4 oder 5, worin die Komponente einen reduzierenden Zucker umfaßt und das Enzym eine entsprechende Zuckeroxidase umfaßt.

7. Verfahren gemäß Anspruch 6, worin die Komponente Glucose umfaßt und das Enzym Glucoseoxidase umfaßt.

8. Vorrichtung zur Durchführung einer elektrochemischen Feststellung oder Messung einer Komponente einer wäßrigen Flüssigkeitsprobe, umfassend:

eine Reaktionszelle mit kapillarer Füllung, die mindestens zwei Elektroden umfaßt, die für die coulometrische Messung geeignet und in einer Reaktionszone oder benachbart dazu angeordnet sind, wobei die Reaktionszone eine wäßrige Flüssigkeitsprobe, die die interessierende Komponente möglicherweise enthält, aufnehmen kann, und wobei die Elektoden und die Reaktionszone so angeordnet sind, daß Flüssigkeit in der Zone die Elektroden berührt und eine Schicht einer Dicke von weniger als etwa 0,2 mm bildet, die über einer der Elektroden liegt;

und die Zelle auch ein Redox-Reagenz in Form einer festen freisetzbaren Schicht umfaßt, die auf einer inneren Oberfläche der Zelle abgeschieden ist und angeordnet ist, um mit der Probe in Berührung zu kommen, wenn diese in die Zelle und in die Reaktonszone eingeführt wird, wobei das Redox-Reagenz in die Lösung in der wäßrigen Probe in der Zone freigesetzt wird und mit der Komponente direkt oder indirekt unter Bildung einer elektrochemisch oxidierbaren oder reduzierbaren Substanz in der Zone reagiert, wobei die oxidierbare oder reduzierbare Substanz elektrochemisch mit der Elektrode reagieren kann und von dem Redox-Reagenz verschieden ist.

9. Vorrichtung gemäß Anspruch 8, die ferner ein Enzym umfaßt, das angeordnet ist, um mit der Probe in Kontakt zu kommen, wenn diese in die Zelle und in die Reaktionszone eingeführt wird, wobei das Enzym fähig ist, eine direkte oder indirekte Reaktion zwischen der Komponente und dem Redox-Reagenz zu katalysieren.

10. Vorrichtung gemäß Anspruch 9, worin das Enzym als eine freisetzbare Schicht auf einer Oberfläche der Reaktionszelle oder Reaktionszone vorhanden ist, die bei Verwendung von der in die Vorrichtung eingeführten wäßrigen Probe berührt wird.

11. Vorrichtung gemäß Anspruch 9 oder 10, worin das Enzym eine Oxidoreduktase umfaßt.

12. Vorrichtung gemäß Anspruch 11, worin das Enzym Glucoseoxidase umfaßt.

13. Vorrichtung gemäß irgendeinem der Ansprüche 8 bis 12, worin das Redox-Reagenz aus der aus Cyanoferrat(III), Methylenblau, p-Benzochinon, 2,6-Dichlorphenolindophenol, Pyocyanin und Thionin bestehenden Gruppe ausgewählt ist und die elektrochemisch oxidierbare oder reduzierbare Substanz oxidierbar ist und eine reduzierte Form des Redox-Reagenzes umfaßt.

14. Vorrichtung gemäß irgendeinem der Ansprüche 8 bis 13, worin die Elektroden aus der Gold-, Kohlenstoff- und Silberelektroden umfassenden Gruppe ausgewählt sind.